Europäisches Patentamt

European Patent Office

Office européen des brevets

(11) Numéro de publication: **0 214 918**
**A1**

## DEMANDE DE BREVET EUROPEEN

(21) Numéro de dépôt: **86440059.3**

(51) Int. Cl.⁴: **A 61 L 9/12**

(22) Date de dépôt: **10.07.86**

(30) Priorité: **09.08.85 FR 8512311**
**25.10.85 FR 8516013**

(43) Date de publication de la demande:
**18.03.87 Bulletin 87/12**

(84) Etats contractants désignés:
**AT BE CH DE GB IT LI LU NL SE**

(71) Demandeur: **SOCIETE INTERNATIONALE DE FABRICATION ET DE DIFFUSION DE PRODUITS PARFUMES-I.P.P. Société à responsabilité limitée Route de Slitzheim Zone Industrielle Remelfing 57200 Sarreguemines (FR)**

(72) Inventeur: **Wendel, Herbert**
**30, rue Jacques Roth**
**F-57200 Sarreguemines (FR)**

**Wendel, Serge**
**86, rue du Maréchal Foch**
**F-57200 Sarreguemines (FR)**

(74) Mandataire: **Aubertin, François**
**Cabinet Lepage & Aubertin Innovations et Prestations 4, rue de Haguenau**
**F-67000 Strasbourg (FR)**

(54) Diffuseur de fluide liquide volatil.

(57) Un diffuseur de fluide liquide volatil tels que désodorisant, parfum, huile essentielle, insecticide et autres, utilisés notamment dans les enceintes fermées telles que habitacles, véhicules, armoires et analogues et comprenant une enveloppe (2) pourvue d'ouvertures (33, 34) pour le passage des vapeurs émises par le fluide liquide volatil (19), une plaquette (14) en matière plus ou moins spongieuse ou absorbante logée dans ladite enveloppe (2) et un contenant (18) obturé par une membrane perforable (20) et rempli de fluide liquide volatil (19), dont le contenant (18) comporte un corps (17) pourvu de moyens de perforation (30, 31) de la membrane perforable (20) et de moyens de prélèvement d'une dose donnée du fluide liquide volatil pour introduire ladite dose, sous l'effet d'au moins une pression exercée par l'usager sur le corps (17), dans la plaquette en matière plus ou moins spongieuse ou absorbante (14).

FIG.2

**Description**

Diffuseur de fluide liquide volatil.

L'invention concerne un diffuseur de fluide liquide volatil tels que désodorisant, parfum, huile essentielle, insecticide et autres, utilisés notamment dans les enceintes fermées telles que habitacles, véhicules, armoires et analogues et comprenant une enveloppe pourvue d'ouvertures pour le passage des vapeurs émises par le fluide liquide volatil, une plaquette en matière plus ou moins spongieuse ou absorbante logée dans ladite enveloppe et un contenant obturé par une membrane perforable et rempli de fluide liquide volatil.

Fréquemment, dans les véhicules automobiles ou dans les moyens de transport en commun tels que autocars, autobus, métro, wagons de chemins de fer, avions et autres, les usagers sont incommodés par les effluves malodorants, les mauvaises odeurs, l'émanation des gaz d'essence, de carburant, etc.... Pour remédier à ces inconvénients, on a conçu des diffuseurs répandant une odeur agréable dans des habitacles divers et, plus particulièrement, dans les véhicules automobiles.

Un tel diffuseur est connu par le document FR-A-1 316 894. Ce diffuseur comporte une plaquette en matière plus ou moins spongieuse ou absorbante logée dans une enveloppe. Celle-ci présente une ouverture à travers laquelle fait saillie une ampoule contenant du parfum, l'embase de cette dernière étant en contact avec la plaquette. Ladite ampoule présente une forme semi-sphérique. Par l'intermédiaire d'une aiguille, on perfore l'embase de l'ampoule et le parfum imbibe la plaquette. Le parfum s'évapore à travers des ouvertures pratiquées dans l'enveloppe. Ledit diffuseur peut être accroché et comporte à cet effet une chaînette.

Cependant, ce diffuseur présente plusieurs inconvénients. En premier lieu, il est nécessaire que l'usager ait à sa disposition une aiguille pour perforer l'embase de l'ampoule semi-sphérique. De plus, avant de perforer l'embase, il est obligé de perforer la plaquette. De ce fait, on risque qu'une partie des parfums s'échappe vers l'extérieur à travers la perforation pratiquée dans la plaquette. Toutefois, l'inconvénient majeur de ce diffuseur réside dans l'écoulement du parfum. En effet, ce diffuseur est disposé verticalement. Si, pour une raison quelconque, l'usager perfore l'embase à proximité de l'extrémité inférieure de l'enveloppe, tout le parfum s'écoule en une seule fois. Ainsi, le diffuseur est prématurément vide et on risque que l'odeur du parfum devienne trop forte et incommode l'usager.

La présente invention a pour but de remédier à ces inconvénients. L'invention, telle qu'elle est caractérisée dans les revendications, résout le problème consistant à créer un diffuseur de fluide liquide volatil tels que désodorisant, parfum, huile essentielle, insecticide et autres, utilisés notamment dans les enceintes fermées telles que habitacles, véhicules, armoires et analogues et comprenant une enveloppe pourvue d'ouvertures pour le passage des vapeurs émises par le fluide liquide volatil, une plaquette en matière plus ou moins spongieuse ou absorbante logée dans ladite enveloppe et un contenant obturé par une membrane perforable et rempli de fluide liquide volatil, dont le contenant comporte un corps pourvu de moyens de perforation de la membrane perforable et de moyens de prélèvement d'une dose donnée du fluide liquide volatil pour introduire ladite dose, sous l'effet d'au moins une pression exercée par l'usager sur le corps, dans la plaquette en matière plus ou moins spongieuse ou absorbante.

Les avantages obtenus grâce à cette invention consistent essentiellement en ce que le fluide liquide volatil peut présenter une certaine viscosité car, contrairement aux diffuseurs connus où l'imprégnation de la plaquette en matière plus ou moins spongieuse ou absorbante se fait par simple écoulement du fluide renfermé dans le contenant, le transvasement du fluide liquide volatil depuis le contenant vers ladite plaquette est commandé par les pressions successives exercées sur le contenant par l'usager. De plus, il est possible de doser le prélèvement du désodorisant, parfum, huile essentielle, insecticide, en fonction, d'une part, des desiderata de l'usager et, d'autre part, du volume de l'enceinte dans laquelle est utilisé le diffuseur conforme à l'invention. Par ailleurs, en raison de cette possibilité de doser un prélèvement, on augmente la durée d'utilisation dudit diffuseur.

L'invention est exposée ci-après plus en détail à l'aide de dessins représentant seulement des modes d'exécution.

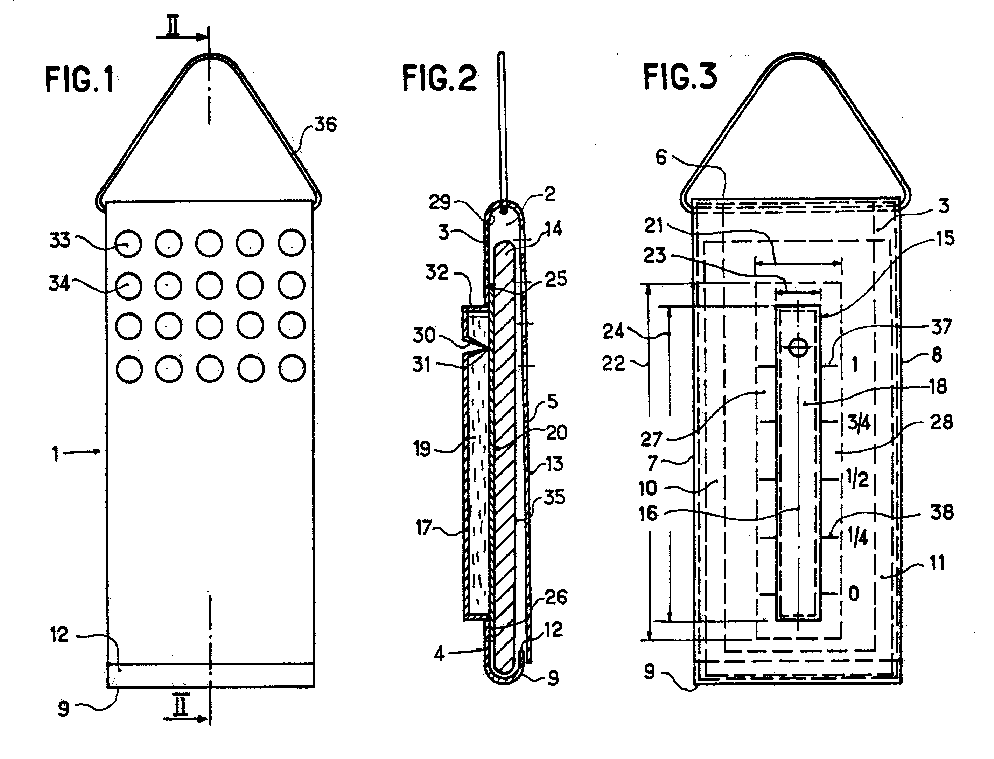

La figure 1 représente en vue en élévation la face arrière du diffuseur conforme à l'invention.

La figure 2 représente une vue en élévation et en coupe selon ligne de coupe II-II de la figure 1.

La figure 3 représente en vue en élévation la face avant dudit diffuseur.

La figure 4 représente une vue en élévation et en coupe, selon ligne de coupe II-II de la figure 1, du diffuseur réalisé selon un autre mode de réalisation.

La figure 5 représente une vue en élévation et en coupe, selon ligne de coupe II-II de la figure 1, du diffuseur réalisé selon un autre mode de réalisation.

Le diffuseur 1 comprend une enveloppe 2 réalisée en un matériau léger tels que carton, feuille d'aluminium et analogue. Cette enveloppe 2 est consituée d'un corps 3 formant la face avant 4 du diffuseur 1 et d'un rabat 5 pivotant autour d'un axe de pliage supérieur 6. On réalise sur les deux chants longitudinaux 7, 8 et sur le chant inférieur 9 des bords recourbés 10, 11, 12 sur lesquels est fixé le rabat 5 par un moyen quelconque, ce rabat 5 constituant la face arrière 13 du diffuseur 1.

On loge dans cette enceinte ainsi formée une plaquette 14. Celle-ci est en matière plus ou moins spongieuse ou absorbante. Cette plaquette 14 occupe pratiquement toute la place disponible à l'intérieur de l'enveloppe 2.

On pratique dans le corps 3 une lumière oblongue 15 dont l'axe longitudinal 16 se confond avec le plan médian du diffuseur 1. A travers cette lumière oblongue 15 passe le corps 17 d'un contenant 18 renfermant un fluide liquide volatil 19, ce corps 17 faisant saillie par rapport à la face avant 4 de l'enveloppe 2. En fonction de l'utilisation du diffuseur 1, ce fluide liquide volatil 19 est un désodorisant, un parfum, une huile essentielle, voire même un insecticide. Conformément à l'invention, le contenant 18 a une forme allongée telle qu'une forme parallélépipédique ou cylindrique dont l'axe longitudinal se confond avec le plan médian du diffuseur 1.

Le contenant 18 est obturé par une membrane 20 dont la largeur 21 et la longueur 22 sont supérieures à la largeur 23 et la longueur 24 de la lumière oblongue 15. De ce fait, les bords supérieur 25 et inférieur 26 ainsi que les bords latéraux 27, 28 viennent prendre appui contre la face interne 29 du corps 3. De ce fait, le contenant 18 est maintenu en place et ne peut sortir hors de la lumière oblongue 15 après assemblage de l'enveloppe 2. Cette membrane 20 prend appui contre la plaquette 14.

Le corps 17 du contenant 18 comporte un élément effilé 30 dont la pointe 31 est pratiquement en contact avec la membrane 20 et faisant saillie par rapport à la face interne dudit corps. Cet élément effilé 30 est destiné à percer la membrane 20 lors de l'utilisation du diffuseur 1. A cet effet, il est nécessaire que la membrane 20 soit constituée d'une feuille imperméable mince facilement perçable telle qu'une feuille en aluminium, une feuille en matière plastique, une feuille en caoutchouc et analogue. Par ailleurs, le corps 17 du contenant 18, réalisé avantageusement en une matière transparente ou au moins translucide, doit être en un matériau élastique. En effet, il faut que l'usager, en exerçant une pression sur le corps 18 à l'emplacement de l'élément effilé 30, puisse enfoncer ce dernier dans la membrane 20 et qu'en relâchant, l'élément effilé 30 libère le trou réalisé dans la membrane 20 pour l'écoulement du fluide liquide volatil 19 pénétrant dans la plaquette 14. Cet élément effilé 30 est situé à proximité du bord supérieur 32 du corps 17 du contenant 18. Ainsi, après perçage, la quantité du fluide liquide volatil 19 pénétrant dans la plaquette 14 est très faible et l'écoulement s'arrête automatiquement lorsque le niveau dudit fluide liquide contenu dans le corps 17 est légèrement en dessous de l'élément effilé 30 dès que le diffuseur 1 est suspendu. Selon un autre mode de réalisation, le niveau du fluide liquide volatil 19 est situé en-dessous de l'élément effilé 30. Ainsi, même si on appuie accidentellement sur le corps 17 et qu'on perce, de ce fait, la membrane 20, aucun fluide liquide volatil 19 pénètre dans la plaquette 14. Ainsi, seuls, le basculement du diffuseur et la pression exercée sur le corps 17 du contenant 18 assurent un écoulement dosé du fluide liquide volatil 19.

Pour faciliter la diffusion de l'odeur, le rabat 5 comporte un certain nombre d'ouvertures 33, 34 traversant de part en part le rabat 5 et débouchant sur la face arrière 35 de la plaquette 14.

Avant l'assemblage de l'enveloppe 2, on met en place un élément d'accrochage 36 permettant de suspendre le diffuseur 1. Cet élément d'accrochage 36 est situé du côté de l'élément effilé 30.

Le cas échéant, on peut pourvoir le corps 3 de repères 37, 38, permettant de connaître la teneur du contenant 18 en fluide liquide volatil 19.

Ainsi, il est parfaitement possible de doser la quantité du fluide volatil 19. En effet, après une première utilisation, c'est-à-dire lorsque le niveau dudit fluide liquide 19 est en-dessous de l'élément effilé 30, il est nécessaire de basculer le diffuseur 1 de sorte que l'élément effilé 30 se trouve en bas. En exerçant une nouvelle pression sur le corps 17 du contenant 18, on prélève une nouvelle quantité du fluide liquide volatil 19 et l'importance de cette quantité est fonction du nombre de pulsions, donc de pressions exercées sur ledit corps 17.

Cependant, il est parfois souhaitable de disposer de diffuseurs de volume important et de les fixer à demeure sur un support tel qu'un mur. De ce fait, il devient impossible de basculer le diffuseur à moins de le démonter, ce qui indispose l'usager.

Par ailleurs, il est parfois difficile de doser les prélèvements du fluide liquide volatil car il n'est pas possible de connaître l'importance du volume de fluide liquide prélevé par impulsion. Ainsi, il peut arriver que le nombre d'impulsions conférées au corps du contenant était trop important, ce qui peut entraîner une diffusion de vapeurs incommodant l'usager.

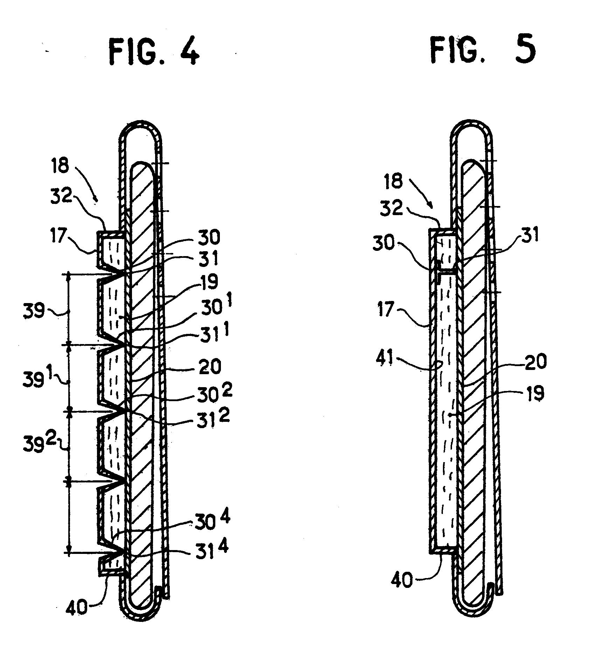

On se réfère à la figure 4.

Le corps de forme allongée 17 est pourvu de plusieurs éléments effilés 30, $30_1$, $30_2$.... Ces différents éléments effilés 30, $30_1$, $30_2$... sont disposés l'un en-dessous de l'autre tout le long du corps 17 du conte nant 18 et sont situés de préférence soit selon une génératrice si le contenant 18 a une forme cylindrique, soit selon l'axe longitudinal si le contenant 18 comporte un corps 17 de forme parallélépipédique.

Il est évident que l'écartement 39 entre deux éléments effilés successifs 30, $30_1$ détermine une dose donnée et prédéterminée. Ainsi, après avoir exercé une première pression sur le corps 17 à l'emplacement de l'élément effilé 30 pour l'écoulement d'une dose de liquide volatil 19 à travers le trou réalisé par la pointe 31 dans la membrane 20, le niveau dudit fluide liquide volatil contenu dans le corps 17 est légèrement en-dessous de la pointe 31 de l'élément effilé 30.

En exerçant une seconde pression sur le corps 17 à l'emplacement de l'élément effilé $30_1$, on pratique dans la membrane 20 un second trou à travers lequel s'écoule une nouvelle dose du fluide liquide volatil 19, celuici s'arrêtant de s'écouler lorsque son niveau sera légèrement en-dessous de la pointe $31_1$ de l'élément effilé $30_1$.

Selon un autre mode d'exécution, les écartements 39, $39_1$, $39_2$... entre les différents éléments effilés 30, $30_1$, $30_2$... sont identiques entre eux. Ainsi, à chaque pression exercée successivement sur les éléments effilés 30, $30_1$, $30_2$..., on prélève et dose identique de fluide liquide volatil 19.

Cependant, en fonction de la nature et du degré de volatilité, il se peut que l'air, situé au-dessus du fluide liquide 19, active la volatilisation de ce dernier et il devient souhaitable que les doses prélevées deviennent de plus en plus importantes. De ce fait, on peut prévoir que, selon un autre mode d'exécu-

tion, les écartements 39, $39_1$, $39_2$ soient progressifs depuis le bord supérieur 32 jusqu'au bord inférieur 40 du corps 17 du contenant 18.

Pour prélever la totalité du fluide liquide volatil 19 contenu dans le corps 17, on peut prévoir que l'élément effilé inférieur $30_4$ comporte une pointe $31_4$ située à proximité du bord inférieur 40 dudit corps 17.

Comme représenté dans la figure 4, le ou les éléments effilés 30, $30_1$, $30_2$... font partie intégrante du corps 17 du contenant 18. Cependant, ces éléments effilés 30, $30_1$, $30_2$... peuvent être formés d'éléments rapportés maintenus en place sur la face interne 41 du corps 17 du contenant 18 par des moyens de fixation quelconque (voir figure 5).

## Revendications

1. Diffuseur de fluide liquide volatil tels que désodorisant, parfum, huile essentielle, insecticide et autres, utilisés notamment dans les enceintes fermées telles que habitacles, véhicules, armoires et analogues et comprenant une enveloppe (2) pourvue d'ouvertures (33, 34) pour le passage des vapeurs émises par le fluide liquide volatil (19), une plaquette (14) en matière plus ou moins spongieuse ou absorbante logée dans ladite enveloppe (2) et un contenant (18) obturé par une membrane perforable (20) et rempli de fluide liquide volatil (19), caractérisé en ce que le contenant (18) comporte un corps (17) pourvu de moyens de perforation (30, 31) de la membrane perforable (20) et de moyens de prélèvement d'une dose donnée du fluide liquide volatil pour introduire ladite dose, sous l'effet d'au moins une pression exercée par l'usager sur le corps (17), dans la plaquette en matière plus ou moins spongieuse ou absorbante (14).

2. Diffuseur de fluide liquide volatil selon la revendication 1, caractérisé en ce que le contenant (18) comporte un corps (17) de forme allongée en matière élastique transparente ou translucide dont l'axe longitudinal se confond avec le plan médian du diffuseur (1) et dont la face interne est pourvue d'au moins un moyen de perforation constitué par un élément effilé (30) faisant saillie par rapport à ladite face interne et pourvu d'une pointe (31) pratiquement en contact avec la membrane perforable (20) et située à proximité du bord supérieur (32) dudit corps (17).

3. Diffuseur de fluide liquide volatil selon la revendication 1, caractérisé en ce que les moyens de perforation (30, 31), faisant saillie par rapport à la face interne du corps (17) du contenant (18), sont situés légèrement en-dessous du fluide liquide volatil (19) renfermé dans le contenant (18) avant l'utilisation du diffuseur (1).

4. Diffuseur de fluide liquide volatil selon la revendication 1, caractérisé en ce que les moyens de perforation (30, 31), faisant saillie par rapport à la face interne du corps (17) du contenant (18), sont situés légèrement au-dessus du fluide liquide volatil (19).

5. Diffuseur de fluide liquide volatil selon la revendication 1, caractérisé en ce que le contenant (18) comporte un corps (17) de forme parallélépipédique.

6. Diffuseur de fluide liquide volatil selon la revendication 1, caractérisé en ce que le contenant (18) comporte un corps (17) de forme cylindrique.

7. Diffuseur de fluide liquide volatil selon la revendication 1, caractérisé en ce que les moyens de perforation sont plusieurs éléments effilés (30, $30_1$, $30_2$...) disposés l'un sous l'autre sur toute la longueur du corps (17) du contenant (18) de sorte que l'élément effilé le plus bas ($30_4$) comporte une pointe ($31_4$) située à proximité du bord inférieur (40) du corps (17) du contenant (18), l'écartement (39, $39_1$, $39_2$...) entre deux éléments effilés successifs ($30, 30_1$ - $30_1, 30_2$...) correspondant à une dose donnée.

8. Diffuseur de fluide liquide volatil selon la revendication 7, caractérisé en ce que les écartements (39, $39_1$, $39_2$...) entre les différents éléments effilés (30, $30_1$, $30_2$) sont identiques.

9. Diffuseur de fluide liquide volatil selon la revendication 1, caractérisé en ce que les écartements (39, $39_1$, $39_2$...) entre les différents éléments effilés (30, $30_1$, $30_2$...) sont progressifs depuis le bord supérieur (32) jusqu'au bord inférieur (40) du corps (17) du contenant (18).

10. Diffuseur de fluide liquide volatil selon la revendication 1, caractérisé en ce que les moyens de perforation (30, 31) font partie intégrante du corps (17) du contenant (18).

**FIG.3**

**FIG.2**

**FIG.1**

FIG. 5

FIG. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | WO-A-8 500 290 (J.P. DESSIMOND) * Page 5, lignes 1-3; revendications 1-4; figure 13 * | 1-10 | A 61 L    9/12 |
| A | FR-A-2 486 402 (O. SATO et al.) * Revendication 7 * | 1 | |
| A | FR-A-1 231 135 (PECHINEY-PROGIL) * Page 1, colonne de droite; figures 3,4 * | 1 | |
| D,A | FR-A-1 316 894 (L.E. PATRIGOT) | 1 | |

DOMAINES TECHNIQUES RECHERCHES (Int. Cl 4)

A 61 L

Le présent rapport de recherche a été établi pour toutes les revendications

| Lieu de la recherche | Date d'achèvement de la recherche | Examinateur |
|---|---|---|
| LA HAYE | 24-11-1986 | PELTRE CHR. |